# EUROPÄISCHE PATENTANMELDUNG

(11) **EP 2 457 610 A1**
(43) Veröffentlichungstag der Anmeldung: **30.05.2012**
(21) Anmeldenummer: 11190343.1
(22) Anmeldetag: 23.11.2011
(51) Int. Cl.: A61M 25/00, A61M 39/00, A61M 39/10, A61M 39/12

(54) **Luer-Lock-Adapter mit Katheteranschluss und Verfahren zur Herstellung eines solchen**

(30) Priorität: 25.11.2010 DE 202010012990 U
(71) Anmelder: RM te me na GmbH, 34587 Felsberg (DE)
(72) Erfinder: Waskönig, Wilhelm, 04720 Aquadulce (Almeria) (ES)
(74) Vertreter: Stoffregen, Hans-Herbert

(57) **Zusammenfassung**

Die Erfindung bezieht sich auf einen Luer-Lock-Adapter (10) mit Katheteranschluss sowie ein Verfahren zur Herstellung eines solchen. Um eine Prallfläche zwischen dem zweiten Schlauchabschnitt und dem innerhalb von diesem verlaufenden Katheterschlauch bei gleichzeitiger Dichtheit der Verbindung auszuschließen, wird vorgeschlagen, dass in dem mit dem Einsatz (22) eine Einheit bildenden zweiten Schlauchabschnitt (24) ein hohlkörperartiger Adapter (30) eingesetzt ist, der öffnungsseitig stetig in den Übergang zwischen dem Innenkegel (20) des Luer-Lock-Adapters (10) und dem Einsatz (22) mit dem zweiten Schlauchabschnitt (24) übergeht und dessen gegenüberliegender gegenüber seinem Eingangsbereichs verjüngter Endabschnitt von einem Abschnitt des Katheterschlauchs (12) mit dem diesen umschließenden eine Einheit bildenden ersten Schlauchabschnitt (16) umgeben ist.

## Beschreibung

Die Erfindung bezieht sich auf einen Luer-Lock-Adapter mit Katheteranschluss, wobei der Katheterschlauch mit einem sich abschnittsweise in dem Luer-Lock-Adapter sich erstreckenden ersten Schlauchabschnitt verbunden ist, der seinerseits innerhalb eines zumindest abschnittsweise in dem Luer-Lock-Adapter sich erstreckenden zweiten Schlauchabschnitt verläuft, der innerhalb eines von dem Luer-Lock-Adapter umspritzten Einsatzes verläuft, wobei Innenkegel des Luer-Lock-Adapters und der Einsatz mit dem zweiten Schlauchabschnitt stufenlos ineinander übergehen und der erste Schlauchabschnitt mit dem Katheterschlauch beanstandet zum Übergang zwischen dem Innenkegel und dem Einsatz verläuft.

Auch nimmt die Erfindung Bezug auf ein Verfahren zur Herstellung eines Luer-Lock-Adapters mit Katheterschlauch.

Entsprechende Luer-Lock-Adapter mit Katheterschlauch werden z. B. für Infusionen für Kleinkinder benötigt. Dabei weist der aus PU (Polyurethan) bestehende Katheterschlauch einen Außendurchmesser im Bereich von 0,4mm auf. Damit ein Abknicken des Katheterschlauchs im Übergang zwischen dem Luer-Lock-Adapter und dem Katheterschlauch unterbleibt, ist dieser seinerseits von einem ersten Schlauchabschnitt umgeben, der sich innerhalb des Luer-Lock-Adapters erstreckt, und von einem vom Inneren des Luer-Lock-Adapters ausgehenden und sich bis außerhalb von diesem erstreckenden zweiten Schlauchabschnitt umgeben ist. Hierdurch ist sichergestellt, dass der Katheterschlauch in Bezug auf den starren Luer-Lock-Adapter im Übergangsbereich hinreichend flexibel ist, ohne dass die Gefahr eines Abknickens des Katheterschlauchs erfolgt.

Der Luer-Lock-Adapter, der aus PA (Polyamid) besteht, wird nach dem Stand der Technik um den auch als Vorspritzling zu bezeichnenden Einsatz gespritzt, in dem seinerseits der zweite Schlauchabschnitt durch Spritzen hergestellt ist. Der zweite Schlauchabschnitt und der Einsatz bestehen üblicherweise aus PU. Der zweite Schlauchabschnitt und der Einsatz gehen mit ihren stirnseitigen Rändern eingangsseitig bündig ineinander über.

Beabstandet zu den eingangsseitigen Stirnrändern des Einsatzes und des zweiten Schlauchabschnitts, die ihrerseits stetig in den Innenkegel des Luer-Lock-Adapters übergehen, verlaufen der Katheterschlauch und der erste Schlauchabschnitt, wobei innenseitiger Stirnrand des ersten Schlauchabschnitts in Bezug auf den stirnseitigen Rand des Katheters zurückversetzt ist. Die aus dem ersten Schlauchabschnitt und dem Katheterschlauch, die beide aus PU bestehen, bestehende Einheit verläuft zurückversetzt zu den stirnseitigen Innenrändern des Einsatzes und des zweiten Schlauchabschnitts, so dass sich zwischen dem Katheter und dem zweiten Schlauchabschnitt ein Ringraum bildet, da der erste Schlauchabschnitt erwähntermaßen zurückversetzt zu dem stirnseitigen Rand des Katheters verläuft. Hierdurch ergibt sich der Nachteil, dass der Fluss der über den Katheterschlauch abzugebenden Infusion gestört wird. Gleichzeitig entsteht eine Undichtigkeit.

Der vorliegenden Erfindung liegt die Aufgabe zugrunde, einen Luer-Lock-Adapter mit Katheteranschluss derart auszubilden, dass eine Prallfläche zwischen dem zweiten Schlauchabschnitt und dem innerhalb von diesem verlaufenden Katheterschlauch mit erstem Schlauchabschnitt ausgeschlossen wird und gleichzeitig die erforderliche Dichtheit zwischen den entsprechenden Elementen erzielbar ist.

Ferner soll ein Verfahren zur Herstellung eines Luerlock-Adapters mit Katheteranschluss derart weitergebildet werden, dass eine flüssigkeitsdichte Verbindung zwischen den Elementen erfolgt, wobei gleichzeitig Bereiche vermieden werden sollen, in denen dem Katheter zuzuführende Flüssigkeit gestaut werden könnte.

Zur Lösung der Aufgabe sieht die Erfindung anordnungsmäßig im Wesentlichen vor, dass in dem mit dem Einsatz eine Einheit bildenden zweiten Schlauchabschnitt ein hohlkörperartiger Adapter eingesetzt ist, dessen Luer-Lock eingangsseitig verlaufender Rand stetig in den Übergang zwischen dem Innenkegel und dem Einsatz mit dem zweiten Schlauchabschnitt übergeht und dessen gegenüberliegender gegenüber seinem Eingangsbereich verjüngter Endabschnitt von einem Abschnitt des Katheterschlauchs mit dem diesen umschließenden und eine Einheit bildenden ersten Schlauchabschnitt umgeben ist.

Erfindungsgemäß wird in den Luer-Lock-Adapter, und zwar in den über den Einsatz mit dem Luer-Lock-Adapter verbundenen zweiten Schlauchabschnitt ein Adapter eingesetzt, der einen stetigen Übergang zu dem zweiten Schlauchabschnitt, dem Einsatz und damit dem Kegel des Luer-Lock-Adapters sicherstellt. Somit kann sich kein von Flüssigkeit beaufschlagter Ringraum bilden. Von dem Adapter selbst geht sodann der Katheterschlauch mit dem diesen umgebenden ersten Schlauchabschnitt aus, so dass weiterhin zwischen dem aus dem Luer-Lock-Adapter austretenden Katheterschlauch und dem Luer-Lock-Adapter Abschnitte des ersten und zweiten Schlauchabschnitts verlaufen, so dass die gewünschte Beweglichkeit des Katheters ohne die Gefahr eines Abquetschens sichergestellt ist.

Der Adapter weist eine trichterförmige Geometrie auf, der einen eine erste Hohlzylindergeometrie aufweisenden Luer-Lock-eingangsseitigen ersten Abschnitt und einen eine zweite Hohlzylindergeometrie aufweisenden ausgangsseitigen zweiten Abschnitt mit gegenüber der ersten Hohlzylindergeometrie kleinerem Innen- und Außendurchmesser umfasst, wobei der Übergang zwischen dem ersten und dem zweiten Abschnitt durch einen Hohlkegel gebildet wird.

Somit ist ein stetiger Übergang von dem Eingangskonus des Luer-Lock-Adapters bis hin zum Eintritt in den Katheterschlauch gewährleistet. Infolgedessen erfolgt eine Störung des Flusses der von dem Luer-Lock-Adapter mit Katheteranschluss durchströmenden Flüssigkeiten nicht. Gleichzeitig ist die erforderliche Dichtigkeit gewährleistet.

Um eine eindeutige Fixierung zwischen dem Adapter und dem Katheterschlauch sicherzustellen - die Verbindung erfolgt insbesondere im Presssitz -, umgibt der Katheter den zweiten Abschnitt bis hin zum Übergang zwischen dem zweiten Abschnitt des Adapters und dem hohlkegelförmigen Abschnitt.

Der zweite Schlauchabschnitt verläuft erwähntermaßen außerhalb des Luer-Lock-Adapters beabstandet zum außenseitig verlaufenden Ende des ersten Schlauchabschnitts und ist mit diesem stoffschlüssig verbunden. Hierzu wird im Übergangsbereich zwischen dem ersten und zweiten Schlauchabschnitt bevorzugterweise ein Lösungsmitteltropfen aufgegeben, der durch Kapillarwirkung in den Zwischenraum zwischen dem ersten und zweiten Schlauchabschnitt eindringt und somit die gewünschte stoffschlüssige Verbindung sicherstellt. Entsprechend kann eine stoffschlüssige Verbindung zwischen dem Katheterschlauch und dem ersten Schlauchabschnitt im Bereich der im Inneren des Luer-Lock-Adapters verlaufenden Enden erfolgen.

Der Adapter selbst wird bevorzugterweise im Presssitz innerhalb des Einsatzes verlaufenden zweiten Schlauchabschnitts fixiert.

Eine bevorzugte Geometrie zwischen den bündig ineinander übergehenden eingangsseitigen Stirnrändern des Einsatzes, des zweiten Schlauchabschnitts und des Adapters ergibt sich dann, wenn der Innenkonus des Luer-Lock-Adapters zu den bündig ineinander übergehenden innen liegenden stirnseitigen Rändern einen stumpfen Winkel α einschließt, der vorzugsweise zwischen 120° und 150° liegt.

Die aus dem Luer-Lock-Adapter mit dem Einsatz und dem zweiten Schlauchabschnitt bestehende Einheit ist des Weiteren in den aus dem Adapter mit dem Katheterschlauch und dem ersten Schlauchabschnitt bestehende Einheit in erstere eingepresst.

Ein Verfahren zur Herstellung eines Luer-Lock-Adapters mit Katheteranschluss mit zumindest einigen der zuvor erläuterten Merkmalen zeichnet sich dadurch aus, dass ein aus dem Luer-Lock-Adapter, dem Einsatz und dem zweiten Schlauchabschnitt bestehende erste Einheit auf die aus dem Adapter, dem Katheterschlauch und dem ersten

Schlauchabschnitt bestehende zweite Einheit zum Einpressen der zweiten in die erste Einheit geschoben wird, wobei der Adapter proximal stetig in Stirnfläche des zweiten Schlauchabschnitts übergeht.

Insbesondere ist vorgesehen, dass zunächst der Einsatz mit dem zweiten Schlauchabschnitt hergestellt wird und sodann an den Einsatz der Luer-Lock-Adapter angespritzt wird.

In Weiterbildung sieht die Erfindung vor, dass vor Verpressen der zweiten Einheit in der ersten Einheit der Katheterschlauch mit dem ersten Schlauchabschnitt im proximalen Bereich stoffschlüssig verbunden wird und/oder dass nach Verpressen der ersten und zweiten Einheit der erste und der zweite Schlauchabschnitt im distalen Bereich stoffschlüssig verbunden werden.

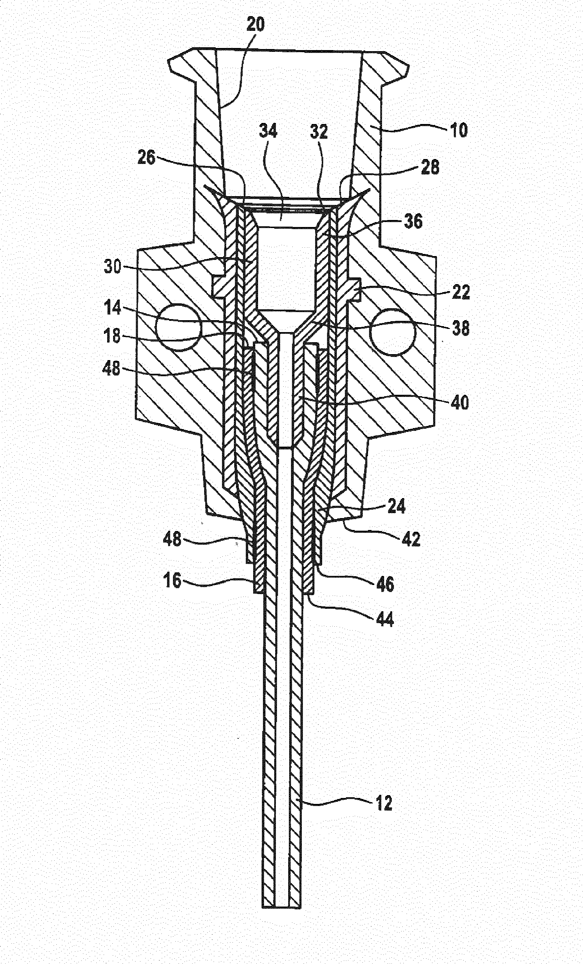

Weitere Einzelheiten, Vorteil und Merkmale der Erfindung ergeben sich nicht nur aus den Ansprüchen, den diesen zu entnehmenden Merkmalen -für sich und/oder in Kombination-, sondern auch aus der nachfolgenden Beschreibung eines der Zeichnung zu entnehmenden bevorzugten Ausführungsbeispiels.

In der einzigen Figur ist eine Schnittdarstellung eines Luer-Lock-Adapters 10 mit einem Abschnitt eines Katheterschlauchs 12 dargestellt. Der Katheterschlauch 12, der z. B. einen Außendurchmesser von 0,35 mm bis 0,4 mm und einen Innendurchmesser von 0,2 mm bis 0,23 mm aufweisen kann, besteht bevorzugterweise aus PU (Polyurethan) und ist Luer-Lock-seitig von einem ersten Schlauchabschnitt 16 umgeben, der gleichfalls aus PU besteht.

Der erste Schlauchabschnitt 16 ist um den endseitigen Abschnitt des Katheters 12 umspritzt und verläuft mit seinem innerhalb des Luer-Lock-Adapters 10 verlaufenden Stirnrand 18 zurückversetzt, also beabstandet zum innen liegenden Stirnrand 20 des Katheterschlauchabschnitts 12.

Der Luer-Lock-Adapter 10 besteht in gewohnter Weise bevorzugterweise aus Polyamid und weist einen Innenkonus 20 auf, in dem ein Einsatz 22 mit innerhalb von diesem verlaufenden zweiten Schlauchabschnitt 24 verläuft. Herstellungstechnisch wird zunächst um den zweiten Schlauchabschnitt der Einsatz 22 gespritzt, die jeweils aus PU bestehen.

Um die so gebildete Einheit wird sodann der Luer-Lock-Adapter 10 gespritzt. Um eine hinreichende Fixierung der stoffschlüssig nicht verbindbaren Materialien PA und PU von Luer-Lock-Adapter 10 und Einsatz 22 zu gewährleisten, weist der Luer-Lock-Adapter 10 innenseitig in gewohnter Weise Vertiefungen auf, in die das PU-Material des Einsatzes 22 eindringt.

Unabhängig hiervon sind die innenseitig verlaufenden Stirnränder 26, 28 des zweiten Schlauchabschnitts 24 und des Einsatzes 22 derart ausgebildet, dass diese bündig ineinander übergehen und zur Innenfläche des Innenkonus des Luer-Lock-Adapters 20 einen stumpfen Winkel einschließen, so dass ein stetiger Übergang gewährleistet ist. Hierzu weisen die stetig ineinander übergehenden Stirnränder 26, 28 zur Innenfläche des Innenkonus 20 einen stumpfen Winkel im Bereich von vorzugsweise zwischen 120° und 150° auf.

Um ohne Störung des Flusses der durch den Luer-Lock-Adapter 10 zum Katheter 12 strömenden Flüssigkeit sicherzustellen, wird in den zweiten Schlauchabschnitt 24 ein Adapter 30 insbesondere aus PA eingesetzt, dessen innenseitig verlaufender Stirnrand 32 stetig in die Stirnränder 26, 28 des Schlauchabschnitts 24 und des Einsatzes 22 übergeht.

Stirnseitig weist der Adapter 30 des Weiteren eine im Schnitt konusförmige Eintrittsöffnung 34 auf, die in einen ersten Hohlzylinderabschnitt 36 des Adapters 30 übergeht, der seinerseits über einen Hohlkegelabschnitt 38 in einen zweiten Hohlzylinderabschnitt 40 übergeht, der in Bezug auf den ersten Hohlzylinderabschnitt 36 sowohl einen kleineren Außen- als auch kleineren Innendurchmesser aufweist, wie die Zeichnung selbsterklärend verdeutlicht.

Auf den zweiten Hohlzylinderabschnitt 30 ist sodann die Einheit erster Schlauchabschnitt 16 und Katheterabschnitt 12 mit ihren Endbereichen aufgeschoben, wobei der Katheterabschnitt 12 sich bis zum Übergang zwischen dem zweiten Hohlzylinderabschnitt und dem Konushohlabschnitt 38 erstreckt.

Auf die so gebildete Einheit wird sodann der Luer-Lock-Adapter 10 mit dem zweiten Abschnitt 24 und dem Einsatz 22 geschoben, wobei eine Verbindung durch Pressen erfolgt.

Wie die zeichnerische Darstellung verdeutlicht, verläuft der erste Schlauchabschnitt 16 im größeren Abstand zum ausgangsseitigen Abschnitt 42 des Luer-Lock-Adapters 10 als der zweite sich ebenfalls über den Luer-Lock-Adapter 10 hinaus erstreckende zweite Schlauchabschnitt 16, so dass sich Stufen 44, 46 bilden.

Die gestuften Übergänge zwischen dem Katheterschlauchabschnitt 12, dem ersten Schlauchabschnitt 16 und dem zweiten Schlauchabschnitt 24 stellen die erforderliche Beweglichkeit des Katheterschlauchs 12 sicher, ohne dass dieser abknicken kann, so dass ein Durchfluss verhindert wäre.

Des Weiteren sollten der erste und zweite Schlauchabschnitt 16, 24 in ihren außerhalb des Luer-Lock-Adapters 10 verlaufenden Bereichen stoffschlüssig miteinander verbunden werden. Hierzu ist bevorzugterweise vorgesehen, dass im Bereich der Stufe 46 ein Lösungsmitteltropfen aufgegeben wird (Bereich 48), der durch Kapillarwirkung in den Zwischenraum zwischen den ersten und zweiten Schlauchabschnitt 16, 24 eindringt und somit ein stoffschlüssiges Verbinden ermöglicht.

Entsprechend wird eine Verbindung zwischen dem Katheterschlauch 12 und dem ersten Schlauchabschnitt 16 im Bereich 48 der innerhalb des Luer-Lock-Adapters verlaufenden Enden hergestellt.

Der erste Schlauchabschnitt 16 sollte insbesondere einen Innendurchmesser zwischen 0,43 und 0,47mm und einen Außendurchmesser zwischen 0,70 und 0,72mm aufweisen. Der zweite Schlauchabschnitt 24 weist bevorzugterweise einen Innendurchmesser von 0,8mm und einen Außendurchmesser von 1,5mm auf.

Durch die diesbezügliche Dimensionierung ist die erforderliche Flexibilität im Ausgangsbereich des Luer-Lock-Adapters 10 für den Katheterschlauch 12 gegeben.

Die Materialien der Elemente sind nicht schutzeinschränkend, sondern nur beispielhaft, wenngleich nach den z. Z. zur Verfügung stehenden Kunststoffen als bevorzugt anzusehen.

## Patentansprüche

1. Luer-Lock-Adapter (10) mit Katheteranschluss, wobei ein Katheterschlauch (12) mit einem sich abschnittsweise in dem Luer-Lock-Adapter sich erstreckenden ersten Schlauchabschnitt (16) verbunden ist, der seinerseits innerhalb eines zumindest abschnittsweise in dem Luer-Lock-Adapter sich erstreckenden zweiten Schlauchabschnitts (24) verläuft, der innerhalb eines von dem Luer-Lock-Adapter umspritzten Einsatzes (22) verläuft, wobei Innenkegel (20) des Luer-Lock-Adapters und der Einsatz mit dem zweiten Schlauchabschnitt stufenlos ineinander übergehen und der erste Schlauchabschnitt mit dem Katheterschlauch beabstandet zum Übergang zwischen dem Innenkegel und dem Einsatz verläuft,
**dadurch gekennzeichnet,**
**dass** in dem mit dem Einsatz (22) eine Einheit bildenden zweiten Schlauchabschnitt (24) ein hohlkörperartiger Adapter (30) eingesetzt ist, der öffnungsseitig stetig in den Übergang zwischen dem Innenkegel (20) des Luer-Lock-Adapters (10) und dem Einsatz (22) mit dem zweiten Schlauchabschnitt (24) übergeht und dessen gegenüberliegender gegenüber seinem Eingangsbereichs verjüngter Endabschnitt von einem Abschnitt des Katheterschlauchs (12) mit dem diesen umschließenden eine Einheit bildenden ersten Schlauchabschnitt (16) umgeben ist.

2. Luer-Lock-Adapter nach Anspruch 1,
**dadurch gekennzeichnet,**
**dass** der Adapter (30) eine trichterförmige Geometrie aufweist.

3. Luer-Lock-Adapter nach Anspruch 1 oder Anspruch 2,
**dadurch gekennzeichnet,**
**dass** der Adapter (30) einen eine erste Hohlzylindergeometrie aufweisenden Luer-Lock-eingangsseitigen ersten Abschnitt (36) und eine eine zweite Hohlzylindergeometrie aufweisenden ausgangsseitigen zweiten Abschnitt (40) mit gegenüber der ersten Hohlzylindergeometrie kleinerem Innen- und Außendurchmesser aufweist, wobei der Übergang zwischen dem ersten und dem zweiten Abschnitt durch einen Hohlkegel gebildet ist.

4. Luer-Lock-Adapter nach zumindest einem der vorhergehenden Ansprüche, **dadurch gekennzeichnet,**
**dass** der Adapter (30) eingangsseitig innenseitig eine Konusform aufweist.

5. Luer-Lock-Adapter nach zumindest einem der vorhergehenden Ansprüche, **dadurch gekennzeichnet,**
**dass** der Katheterschlauch (12) den zweiten Abschnitt (40) des Adapters (30) bis hin zum durch den Hohlkegelabschnitt gebildeten Übergang umgibt.

6. Luer-Lock-Adapter nach zumindest einem der vorhergehenden Ansprüche, **dadurch gekennzeichnet,**
**dass** der Katheterschlauch (24) mit dem vorzugsweise beabstandet zu dem Übergang verlaufenden ersten Schlauchabschnitt (16) den Adapter (30) in Presssitz umgibt.

7. Luer-Lock-Adapter nach zumindest einem der vorhergehenden Ansprüche, **dadurch gekennzeichnet,**
**dass** der zweite Schlauchabschnitt (24) außerhalb des Luer-Lock-Adapters (10) beabstandet zum außenseitig verlaufenden Stirnrand des ersten Schlauchabschnitts (16) verläuft und mit diesem stoffschlüssig verbunden ist.

8. Luer-Lock-Adapter nach zumindest einem der vorhergehenden Ansprüche, **dadurch gekennzeichnet,**
**dass** der Adapter (30) vollständig innerhalb des Luer-Lock-Adapters (10) und beanstandet zu dessen ausgangsseitigem Ende (42) verläuft.

9. Luer-Lock-Adapter nach zumindest einem der vorhergehenden Ansprüche, **dadurch gekennzeichnet,**
**dass** der Adapter (30)im Presssitz in dem innerhalb des Einsatzes (22) verlaufenden zweiten Schlauchabschnitt (24) fixiert ist.

10. Luer-Lock-Adapter nach zumindest einem der vorhergehenden Ansprüche, **dadurch gekennzeichnet,**
**dass** der Innenkonus (20) des Luer-Lock-Adapters (10) zu den bündig ineinander übergehenden Stirnrändern (26, 28) des Einsatzes (22), des zweiten Schlauchabschnitts (24) und des Adapters (30) einen stumpfen Winkel α mit bevorzugterweise 120° ≤ α ≤ 150° einschließt.

11. Luer-Lock-Adapter nach zumindest einem der vorhergehenden Ansprüche, **dadurch gekennzeichnet,**
**dass** der Adapter (30) aus Polyamid besteht.

12. Luer-Lock-Adapter nach zumindest einem der vorhergehenden Ansprüche, **dadurch gekennzeichnet,**
**dass** der Luer-Lock-Adapter (10) mit dem Einsatz (22) und dem zweiten Schlauchabschnitt (24) eine erste Einheit und der Adapter (30) mit dem Katheterschlauch (12) und dem ersten Schlauchabschnitt (16) eine zweite Einheit bilden, die in die erste Einheit eingepresst ist.

13. Verfahren zur Herstellung eines Luer-Lock-Adapters (10) mit Katheterschlauch (12) nach zumindest Anspruch 1,
**dadurch gekennzeichnet,**
**dass** eine aus dem Luer-Lock-Adapter (10), dem Einsatz (22) und dem zweiten Schlauchabschnitt (24) bestehende erste Einheit auf eine aus dem Adapter (30), dem Katheterschlauch und dem ersten Schlauchabschnitt (16) bestehende zweite Einheit zum Einpressen der zweiten Einheit in die erste Einheit geschoben wird, wobei der Adapter proximal in Stirnfläche (26) des zweiten Schlauchabschnitts stetig übergeht.

14. Verfahren nach Anspruch 13,
**dadurch gekennzeichnet,**
**dass** zunächst der Einsatz (22) mit dem zweiten Schlauchabschnitt (24) hergestellt und sodann an den Einsatz der Luer-Lock-Adapter (10) angespritzt wird.

15. Verfahren nach Anspruch 13 oder 14,
**dadurch gekennzeichnet,**
**dass** vor Verpressen der zweiten Einheit in der ersten Einheit der Katheterschlauch (12) mit dem ersten Schlauchabschnitt (24) im proximalen Bereich stoffschlüssig verbunden werden und/oder dass nach Verpressen der ersten und der zweiten Einheit der erste und der zweite Schlauchabschnitt (16, 24) im distalen Bereich des zweiten Schlauchabschnitts stoffschlüssig verbunden werden.
